(19) **Europäisches Patentamt**
**European Patent Office**
**Office européen des brevets**

(11) **EP 4 553 063 A1**

(12) **EUROPEAN PATENT APPLICATION**
published in accordance with Art. 153(4) EPC

(43) Date of publication:
**14.05.2025 Bulletin 2025/20**

(21) Application number: **23835511.9**

(22) Date of filing: **04.07.2023**

(51) International Patent Classification (IPC):
**C07C 63/307** *(2006.01)* **C07F 1/08** *(2006.01)*

(52) Cooperative Patent Classification (CPC):
**C07C 63/307; C07F 1/08**

(86) International application number:
**PCT/JP2023/024708**

(87) International publication number:
**WO 2024/009979 (11.01.2024 Gazette 2024/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.07.2022 JP 2022107626**

(71) Applicant: **Mitsui Mining & Smelting Co., Ltd.**
**Shinagawa-ku**
**Tokyo 141-8584 (JP)**

(72) Inventors:
- **MASAKI, Takanori**
  **Ageo-shi, Saitama 362-0021 (JP)**
- **MATSUMAE, Kazuo**
  **Ageo-shi, Saitama 362-0021 (JP)**
- **SENOO, Yuichi**
  **Ageo-shi, Saitama 362-0021 (JP)**
- **KODAIRA, Makoto**
  **Ageo-shi, Saitama 362-0021 (JP)**

(74) Representative: **Novagraaf Technologies**
**2 rue Sarah Bernhardt**
**CS90017**
**92665 Asnières-sur-Seine Cedex (FR)**

(54) **METAL-ORGANIC STRUCTURE, AND METHOD FOR MANUFACTURING SAME**

(57) Provided is a metal organic framework having a volume mean particle diameter of from 1.0 to 30.0 $\mu$m, and a ratio $D_{10}/D_{90}$, which is a ratio of a median diameter $D_{10}$ to a median diameter $D_{90}$, of from 0.35 to 1.00. The metal organic framework can suitably be manufactured by a method for manufacturing a metal organic framework involving a centrifuging-shearing step of simultaneously applying centrifugal force and shearing force to a blend including a metal ion donor, a multidentate ligand, and a solvent, wherein the method further involves a step of recovering the blend subjected to the centrifuging-shearing step and subjecting the recovered blend to the centrifuging-shearing step again, or wherein the median diameter $D_{50}$ of the multidentate ligand is from 0.1 to 50.0 $\mu$m.

**Fig. 1**

**EP 4 553 063 A1**

## Description

**Technical Field**

The present invention relates to a metal organic framework and a method for manufacturing the same.

**Background Art**

Recent years have seen increasing use of various gases having a smaller environmental impact than petroleum, in an effort toward decarbonization. Against this backdrop, substances called metal organic frameworks (MOFs), or porous coordination polymers (PCPs), are drawing attention in such fields as gas storage and gas separation. Metal organic frameworks are compounds having a structure in which metal atoms are linked via organic ligands, and are typically porous.

Typical examples of methods for manufacturing metal organic frameworks include liquid-phase synthesis methods such as solution-stirring, hydrothermal synthesis, solvothermal synthesis, microwave irradiation, electrolytic synthesis, flow reactor synthesis, spray-drying synthesis, ultrasonic irradiation, etc. There are also cases where solid-phase synthesis, using a mortar, a ball mill device, etc., is employed. Further, in recent years, there have also been reports of methods for synthesizing metal organic frameworks by employing a biaxial mixing/kneading device called an extruder. Unfortunately, when employing the aforementioned methods, there are cases where it is difficult to synthesize high-quality metal organic frameworks in a short reaction time.

Patent Literature 1 proposes a method for manufacturing a high-quality MOF in a short time by applying centrifugal force and shearing force to a blend obtained by mixing a metal ion donor, a multidentate ligand, and a solvent as raw materials.

Citation List

Patent Literature

Patent Literature 1: EP 3971160 A1

**Summary of Invention**

Various methods have been proposed as methods for employing manufactured MOFs, with examples including methods of employing particulate MOF as a raw material and processing it into compacts, such as pellets or sheets, to be used as adsorbents etc. Thus, there has been a demand for MOFs having better processability. When processing particulate MOF into a compact, it is preferable that the MOF is constituted by fine particles from the viewpoint of processability. Further, to improve packability when being processed into a compact, it is preferable that the MOF is monodisperse and has high flowability. Therefore, the MOF manufactured according to the conventional method disclosed in Patent Literature 1 still has room for improvement in terms of monodispersibility and flowability.

An objective of the present invention is to provide MOF particles which are constituted by fine particles, have a sharp particle size distribution, and have excellent monodispersibility and flowability.

As a result of diligent study to achieve the aforementioned objective, Inventors have found that MOF particles constituted by fine particles and having a sharp particle size distribution can be manufactured by repeatedly applying centrifugal force and shearing force, or by particulating, in advance, a multi dentate ligand which serves as a raw material and applying centrifugal force and shearing force thereto.

The present invention provides a metal organic framework having

a volume mean particle diameter of from 1.0 to 30.0 $\mu$m, and
a ratio $D_{10}/D_{90}$, which is a ratio of a median diameter $D_{10}$ to a median diameter $D_{90}$, of from 0.35 to 1.00.

The present invention also provides a method for manufacturing a metal organic framework, involving

a centrifuging-shearing step of simultaneously applying centrifugal force and shearing force to a blend including a metal ion donor, a multidentate ligand, and a solvent, and
a step of recovering the blend subjected to the centrifuging-shearing step and subjecting the recovered blend to the centrifuging-shearing step again.

The present invention also provides a method for manufacturing a metal organic framework, involving a centrifuging-shearing step of simultaneously applying centrifugal force and shearing force to a blend including a metal ion donor, a multidentate ligand, and a solvent, wherein
a median diameter $D_{50}$ of the multidentate ligand is from 0.1 to 50.0 $\mu$m.

**Brief Description of Drawings**

[Fig. 1] Fig. 1 is graph showing an X-ray diffraction (XRD) pattern of a MOF obtained in Example 3.
[Fig. 2] Fig. 2 is graph showing an X-ray diffraction (XRD) pattern of a MOF obtained in Comparative Example 2.
[Fig. 3] Fig. 3 is graph showing an X-ray diffraction (XRD) pattern of a MOF obtained in Example 11.

**Description of Embodiments**

The present invention is described below according to preferred embodiments thereof.

A metal organic framework (also abbreviated as "MOF" hereinbelow) of the present invention has a volume mean particle diameter of from 1.0 to 30.0 $\mu$m, and a ratio $D_{10}/D_{90}$, which is ratio of a median diameter $D_{10}$ to a median diameter $D_{90}$, of from 0.35 to 1.00. When a MOF possesses these properties, the MOF particles will have excellent monodispersibility and excellent flowability. Further, since the MOF is particulated (i.e., made into fine particles), when producing pellets or other structures at a later stage, the size of such structures can be controlled flexibly.

From the viewpoint of handleability at the time of processing particulated MOF particles into a compact, it is preferable that the volume mean particle diameter is preferably 1.5 $\mu$m or greater, more preferably 2.0 $\mu$m or greater, even more preferably 2.5 $\mu$m or greater, most preferably 3.0 $\mu$m or greater. From the same viewpoint, it is preferable that the volume mean particle diameter is preferably 20.0 $\mu$m or less, more preferably 15.0 $\mu$m or less, even more preferably 10.0 $\mu$m or less, further preferably 8.0 $\mu$m or less, most preferably 6.0 $\mu$m or less.

From the viewpoint of monodispersibility and flowability, it is preferable that the ratio $D_{10}/D_{90}$, which is a ratio of a median diameter $D_{10}$ to a median diameter $D_{90}$, is preferably from 0.37 to 1.00, more preferably from 0.40 to 1.00. As the ratio $D_{10}/D_{90}$ approaches 1, the particle size distribution of the MOF becomes sharp, and thus, not only is monodispersibility improved, but also flowability.

The volume mean particle diameter, $D_{10}$, and $D_{90}$ can be determined, for example, by laser diffraction/scattering method particle size distribution measurement. "Volume mean particle diameter" is a value determined by laser diffraction/scattering method particle size distribution measurement. Assume that there is a group of powder body, and in that group, there are particles respectively having particle diameters of d1, d2, ... , di, ... , dk in ascending order of particle diameter, and there are respectively n1, n2, ... , ni,..., nk pieces of such particles. Here, the surface area of a single particle is defined as ai, and the volume is defined as vi. In this case, the volume mean particle diameter MV, weighted by volume, is calculated according to the following equation (cf. instruction manual for Microtrac (MT3000) from MicrotracBEL Corp.). $D_{10}$ and $D_{90}$ are respectively the cumulative volume particle diameters at a cumulative volume of 10 vol% and 90 vol% as measured according to laser diffraction/scattering method particle size distribution measurement.

$$MV = (v1{\times}d1+v2{\times}d2+...+vi{\times}di+...+vk{\times}dk)/(v1+v2+...+vi+...vk)$$

From the viewpoint of particulation, it is preferable that, in the MOF of the present invention, the volume frequency of particles having a particle diameter of less than 2 $\mu$m is preferably 20% or less, more preferably 15% or less, even more preferably 10% or less. By satisfying this requirement, high flowability can be secured. The lower limit of the volume frequency is not particularly limited, but is typically 0% or greater.

It is preferable that the MOF of the present invention has an angle of rest of 50° or less, more preferably 45° or less, even more preferably 40° or less. When the angle of rest has a small value as described above, high flowability of the MOF can be secured. The lower limit of the angle of rest is not particularly limited, but is typically 0° or greater.

Note that the "angle of rest" refers to the angle between the horizontal plane and a sloping surface of a heap formed by MOF which stably comes to rest on its own without collapsing after being caused to free fall from a given height.

The angle of rest of the MOF of the present invention can be measured with an angle-of-rest measurement device constituted by a funnel, a measurement stage, etc., by causing the MOF to free fall and measuring the angle of the powder body formed after falling. More specifically, measurement can be conducted by, for example: pouring a sample onto a measurement stage (measurement stage diameter: 30 mm) from a funnel (funnel hole diameter: 5 mm; funnel angle: 60°) at a height of 40 mm; while dropping the sample toward the measurement stage from a height of 40 mm, ceasing to pour the sample when the sample overflows from the measurement stage and falls out to the periphery; and measuring the angle between the measurement stage and the powder body with a protractor three times, to find the average value thereof as the measurement value.

Further, it is preferable that the MOF of the present invention has a compression degree of preferably 50% or less, more preferably less than 45%, even more preferably 40% or less, most preferably 35% or less. "Compression degree" is a value found by dividing the difference between aerated bulk density and packed bulk density by the packed bulk density. When the flowability is high, the difference between aerated bulk density and packed bulk density tends to become small and the compression degree tends to become small. Since the compression degree of the MOF of the present invention has a low value as described above, high flowability of the MOF can be secured.

The compression degree can be calculated by dividing the difference between aerated bulk density and packed bulk density by the packed bulk density.

The aerated bulk density can be found by dropping the MOF from a funnel into a cup having a discretionary volume, scraping off excessive amounts of the MOF, and measuring the weight. The packed bulk density can be found by measuring the volume and weight of powder after it has been tapped a plurality of times under a given condition until the volume of the powder body inside the cup reaches a given value. More specifically, the aerated bulk density can be found, for example, by: dropping a sample from a funnel (funnel hole diameter: 5 mm, funnel angle: 60°) at a height of 40 mm into a cup (volume: 25 cm$^3$) until the sample overflows from the cup; horizontally scraping off excessive amounts of the sample overflowing from the cup by using a metal spatula; calculating the density by dividing the measured weight of the sample inside the cup by the cup volume (25 cm$^3$); and measuring the density three times, to find the average value thereof as the aerated bulk density.

The packed bulk density can be found, for example, by: placing 5 g of a sample in a graduated cylinder; conducting tapping repeatedly from a height of 4 cm until the graduated position of the powder body reaches a given level; and dividing the sample weight, measured at the time the graduated position reaches a given level, by the sample volume observed from the graduation, to calculate the packed bulk density.

It is preferable that the MOF of the present invention has a BET specific surface area of preferably from 100 to 10000 m$^2$/g, more preferably from 200 to 5000 m$^2$/g, even more preferably from 500 to 4000 m$^2$/g, further preferably from 1000 to 2300 m$^2$/g, further preferably from 1650 to 2300 m$^2$/g, most preferably from 1700 to 2300 m$^2$/g. When the MOF has a high specific surface area as described above, the MOF can exhibit excellent properties in various applications; for example, when used for gas storage, a large amount of gas can be stored in the MOF.

The MOF includes a metal ion donor and a multidentate ligand.

Examples of metal elements constituting the metal ion donor may include discretionary elements belonging to alkali metals (Group 1), alkaline-earth metals (Group 2), transition metals (Groups 3 to 12), Group 13 elements, and rare-earth elements (atomic numbers 21, 29, and 57 to 71). Typical examples of the metal element may include at least one selected from copper, iron, zinc, zirconium, aluminum, cobalt, magnesium, nickel, chromium, hafnium, and lanthanum. That is, the metal ion donor includes at least one type of metal ion selected from copper ions, iron ions, zinc ions, zirconium ions, aluminum ions, cobalt ions, magnesium ions, nickel ions, chromium ions, hafnium ions, and lanthanum ions, which are ions of the aforementioned metal elements.

The metal ion is typically hexa-coordinated or tetra-coordinated. In cases where the metal ion is hexa-coordinated, it is preferable to use a bidentate ligand as the multidentate ligand (described below) from the viewpoint of successfully synthesizing the MOF. From the same viewpoint, in cases where the metal ion is tetra-coordinated, it is preferable to use a tridentate ligand as the multi dentate ligand (described below).

For the metal ion donor, a metal salt is typically used. The metal ion donor may be an organic salt or an inorganic salt. The metal ion donor is typically selected from the group consisting of hydroxide salts, carbonate salts, acetate salts, sulfate salts, nitrate salts, and chloride salts. It is also possible to use, in combination, a plurality of metal ion donors containing the same metal element.

The multidentate ligand is typically an organic multidentate ligand, and may be selected, for example, from the group consisting of carboxylic acid anions, amine compounds, sulfonic acid anions, phosphoric acid anions, and heterocyclic compounds. The carboxylic acid anion may be, for example, an anion of at least one organic compound selected from trimesic acid (BTC), terephthalic acid (BDC), isophthalic acid, 2-methylimidazole, 2-5-dihydroxyterephthalic acid (dobdc or DHTA), 4,4'-dioxido-3,3'-biphenyldicarboxylate (dobpdc), fumaric acid, formic acid, 1,3,5-tris(4-carboxyphenyl)benzene (BTB), 4,4'-biphenyldicarboxylic acid (BPDC), oxalic acid, 1,2,4-triazole, and 3,5-pyrazole dicarboxylic acid (H3PDC). In this case, by combining the aforementioned metal ion and the aforementioned multidentate ligand, it is possible to produce MOFs having various properties, such as biocompatibility, catalytic properties, photoresponsiveness, semiconducting properties, etc.

Combinations of metal ions and multidentate ligands to be included in MOFs of the present invention, as well as MOFs including such combinations, are not particularly limited, and preferable examples may include Al(OH)(bpdc) including Al and BPDC, CAU-10 including Al and isophthalic acid, CAU-4 including Al and BTB, MIL-100Al including Al and BTC, MIL-53Al including Al and BDC, MIL-53-Fum including Al and fumaric acid, MOF-303 including Al and H3PDC, ZIF-67 including Co and 2-methylimidazole, MOF-74Co including Co and dopdc, MIL-101Cr including Cr and BDC, Cu(H$_2$)(1,3-BDC) including Cu and isophthalic acid, MOF-199 including Cu and BTC, MIL-100Fe including Fe and BTC, MIL-53Fe including Fe and BDC, MIL-88B including Fe and BDC, La-BTB including La and BTB, MOF-274 including Mg and dopdc, MOF-74Mg including Mg and dopdc, Mg-formate including Mg and formic acid, CALF-20 including Zn and oxalic acid, MOF-177 including Zn and BTB, MOF-5 including Zn and BDC, MOF-74Zn including Zn and dopdc, ZIF-8 including Zn and 2-methylimidazole, MOF-801 including Zr and fumaric acid, UiO-66 including Zr and BDC, and MOF-74Ni including Ni and dopdc.

Next, preferable methods for manufacturing MOFs of the present invention will be described.

First, a blend including a metal ion donor, a multidentate ligand, and a solvent is prepared. For the metal ion donor and the multidentate ligand, the aforementioned materials are used. The solvent is not particularly limited, and it is possible to use a solvent generally used for synthesizing metal organic frameworks. Note, however, that the solvent is preferably a poor solvent to at least one of the metal ion donor and the multidentate ligand. With this composition, the blend does not completely become a solution, but becomes semi-solid, and typically a slurry, with solid components remaining therein. In this way, the later-described centrifugal force and shearing force can be applied more effectively to the blend.

Herein, the expression, a solvent is a "poor solvent" in relation to a certain object, means that the solubility of the object to the solvent is less than or equal to 1 g/50 mL (=20 g/L) at 25°C and atmospheric pressure. Examples of usable solvents may include water, alcohols such as methanol, ethanol, etc., carboxylic acids such as formic acid, acetic acid, etc., amides such as N,N-dimethylformamide (DMF), N,N-diethylformamide (DEF), etc., and esters such as ethyl acetate etc. A plurality of solvents may be used as a mixture.

The amount of the solvent may be, for example, 100 mass% or greater, preferably 200 mass% or greater, with respect to the total amount of the metal ion donor and the multi dentate ligand.

Further, the amount of the solvent may be, for example, 1000 mass% or less, preferably 700 mass% or less, even more preferably 500 mass% or less, with respect to the total amount of the metal ion donor and the multidentate ligand.

With this composition, for example, it is possible to improve MOF manufacturing efficiency, and in addition, in cases where an organic solvent is used, it is possible to reduce environmental load by reducing the amount of solvent used.

The blend may further include additional substances such as reaction accelerators etc. The reaction accelerator is, for example, a basic substance or an acidic substance, and is typically a basic substance. Examples of the basic substance may include diethylamine, triethylamine, 2,6-lutidine, pyridine, imidazole, potassium hydroxide, and sodium hydroxide. Examples of the acidic substance may include formic acid, acetic acid, trifluoroacetic acid, sulfuric acid, nitric acid, hydrochloric acid, and phosphoric acid. A plurality of reaction accelerators may be used in combination as additional substances. A reaction control agent may be added as an additional substance.

The blend is mixed while applying centrifugal force and shearing force simultaneously and continuously. The mixture obtained by this mixing is recovered, and is mixed again while applying centrifugal force and shearing force. In this way, a high-quality MOF can be manufactured in a short time, and a MOF having monodispersibility and high flowability as described above can be obtained successfully.

An example of a method for applying centrifugal force and shearing force simultaneously and continuously is described below. First, a blend is introduced into a reaction vessel. Next, by rotating a rotary blade provided in the reaction vessel, the blend is stirred at high speed. By this rotation operation, centrifugal force is applied to the blend, and the blend is pressed against the inner wall of the reaction vessel. In this way, the blend is brought into contact with the inner wall of the reaction vessel, and thus, both centrifugal force and shearing force are applied simultaneously and continuously to the blend. In this state, high energy by the shearing force is applied to the blend, and the metal ion donor and the multidentate ligand in the blend undergo reaction, thereby obtaining a MOF. The aforementioned shearing force may also occur due to mutual contact among particles constituting the blend.

An example of the aforementioned method includes thin-film spin mixing developed by PRIMIX Corporation. In this method, a thin-film spin-type high-speed mixer can be used to apply centrifugal force and shearing force simultaneously and continuously to a substance that has been introduced. Specific device configurations are disclosed in JP2007-125454A, for example.

Examples of methods for recovering the mixture obtained according to the aforementioned method and mixing the same while applying centrifugal force and shearing force again may include: a batch method wherein the mixture is, literally, recovered from the mixer and re-introduced into the mixer again; and a circulating method (continuous method) wherein the mixture is recovered from the mixer which is provided with a circulation pipe and a pump, and continuously re-introducing the recovered mixture to the mixer through the circulation pipe.

In cases of re-introducing the recovered mixture into the mixer again and applying centrifugal force and shearing force thereto again, the number of times the step of applying centrifugal force and shearing force is conducted is 2 times or more, preferably from 3 to 10 times, more preferably from 3 to 8 times, even more preferably from 3 to 5 times. In this way, the MOF of the present invention, having excellent monodispersibility and flowability as described above, can be obtained successfully. In cases where recovery of the mixture is conducted according to the circulating method (continuous method), the aforementioned "number of times" refers to the number of times calculated by dividing the time that the mixer is in operation by the time that slurry passes through the reaction vessel once. The "time that slurry passes through the reaction vessel once" can be calculated by dividing the slurry's volume (L) by the slurry's flow speed (L/min).

The time that centrifugal force and shearing force are applied to the mixture is not particularly limited in both the batch method and the circulating method (continuous method) and can be adjusted as appropriate depending on the reaction vessel's volume, the mixture's volume, the slurry's flow speed, and the rotary blade's circumferential speed.

Further, in the method for manufacturing the MOF of the present invention, when mixing the blend while applying centrifugal force and shearing force simultaneously and continuously, the raw material may be particulated (made into fine particles) in advance instead of, or in addition to, recovering the obtained mixture and again mixing the recovered mixture while applying centrifugal force and shearing force. Also in this way, a MOF having monodispersibility and high flowability as described above can be obtained successfully.

In this case, the median diameter $D_{50}$ of the multidentate ligand in the aforementioned blend is preferably 0.1 $\mu$m or greater, more preferably 1.0 $\mu$m or greater, even more preferably 5.0 $\mu$m or greater, further preferably 10.0 $\mu$m or greater, particularly preferably 20.0 $\mu$m or greater.

Further, the median diameter $D_{50}$ of the multidentate ligand is preferably 130 $\mu$m or less, more preferably 80.0 $\mu$m or less, even more preferably 50.0 $\mu$m or less, further preferably 40.0 $\mu$m or less, particularly preferably 30.0 $\mu$m or less.

The median diameter $D_{50}$ of the metal ion donor is not particularly limited, and may be, for example, preferably 1.0 $\mu$m or greater, more preferably 5.0 $\mu$m or greater, even more preferably 10.0 $\mu$m or greater.

Further, the median diameter $D_{50}$ of the metal ion donor may be, for example, preferably 50.0 $\mu$m or less, more preferably 30.0 $\mu$m or less, even more preferably 20.0 $\mu$m or less.

"Median diameter" refers to the cumulative volume particle diameter at a cumulative volume of 50 vol% as measured according to laser diffraction/scattering method particle size distribution measurement. For example, this can be determined according to laser diffraction/scattering method particle size distribution measurement in the same way as for the volume mean particle diameter, $D_{10}$, and $D_{90}$ of the MOF.

In cases of manufacturing the MOF by using particulated raw material as described above, the number of times the step of applying centrifugal force and shearing force is conducted is preferably from 1 to 10 times, more preferably from 2 to 10 times, even more preferably from 3 to 10 times, most preferably from 3 to 5 times. In this way, bulky raw material can be pulverized in advance, and thus, the MOF of the present invention, which has excellent monodispersibility and flowability as described above, can be obtained successfully. The "number of times" can be calculated in the same manner as described above.

Further, in the aforementioned manufacturing method, it is preferable that, in either one of the two methods described above, the circumferential speed of the rotary blade is preferably from 10 to 100 m/s, more preferably from 20 to 80 m/s, even more preferably from 20 to 60 m/s, further preferably from 20 to 40 m/s. In this way, the MOF of the present invention, which has excellent monodispersibility and flowability as described above, can be obtained successfully.

The present invention has been described above according to preferred embodiments thereof, but the present invention is not limited to the foregoing embodiments.

The present invention encompasses the following embodiments.

{1} A metal organic framework having a volume mean particle diameter of from 1.0 to 30.0 $\mu$m, and a ratio $D_{10}/D_{90}$, which is a ratio of a median diameter $D_{10}$ to a median diameter $D_{90}$, of from 0.35 to 1.00.

{2} The metal organic framework as set forth in clause {1}, wherein a volume frequency of particles having a particle diameter of less than 2 $\mu$m is 20% or less.

{3} The metal organic framework as set forth in clause {1} or {2}, having an angle of rest of 50° or less.

{4} The metal organic framework as set forth in any one of clauses {1} to {3}, having a compression degree of 50% or less.

{5} The metal organic framework as set forth in any one of clauses {1} to {4}, including at least one of metal ion selected from alkali metal ions, alkaline-earth metal ions, transition metal ions, Group 13 element ions, and rare-earth element ions.

{6} The metal organic framework as set forth in clause {5}, wherein the metal ion includes at least one of metal ion selected from a copper ion, an iron ion, a zinc ion, a zirconium ion, an aluminum ion, a cobalt ion, a magnesium ion, a calcium ion, a nickel ion, a chromium ion, a hafnium ion, and a lanthanum ion.

{7} The metal organic framework as set forth in any one of clauses {1} to {6}, including at least one of organic compound selected from trimesic acid (BTC), terephthalic acid (BDC), isophthalic acid, 2-methylimidazole, 2-5-dihydroxyterephthalic acid (dobdc or DHTA), 4,4'-dioxido-3,3'-biphenyldicarboxylate (dobpdc), fumaric acid, formic acid, 1,3,5-tris(4-carboxyphenyl)benzene (BTB), 4,4'-biphenyldicarboxylic acid (BPDC), oxalic acid, 1,2,4-triazole, and 3,5-pyrazole dicarboxylic acid (H3PDC).

{8} The metal organic framework as set forth in any one of clauses {1} to {7}, having a BET specific surface area of from 100 to 10000 m$^2$/g.

{9} A method for manufacturing a metal organic framework, involving

a centrifuging-shearing step of simultaneously applying centrifugal force and shearing force to a blend including a metal ion donor, a multidentate ligand, and a solvent, and

a step of recovering the blend subjected to the centrifuging-shearing step and subjecting the recovered blend to the centrifuging-shearing step again.

{10} The method for manufacturing a metal organic framework as set forth in clause {9}, wherein a number of times the centrifuging-shearing step is conducted is from 3 to 10 times.

{11} A method for manufacturing a metal organic framework, involving a centrifuging-shearing step of simultaneously applying centrifugal force and shearing force to a blend including a metal ion donor, a multidentate ligand, and a solvent,
wherein a median diameter $D_{50}$ of the multidentate ligand is from 0.1 to 50.0 $\mu$m.

{12} The method for manufacturing a metal organic framework as set forth in clause {11}, wherein a number of times the centrifuging-shearing step is conducted is from 1 to 10 times.

{13} The method for manufacturing a metal organic framework as set forth in any one of clauses {9} to {12}, wherein

the centrifuging-shearing step uses a reaction vessel including a rotary blade, and
a circumferential speed of the rotary blade is from 10 to 100 m/s.

**Examples**

Examples 1 and 2:

Copper hydroxide ($Cu(OH)_2$) was used as a metal ion donor, trimesic acid (BTC) was used as a multidentate ligand, methanol was used as a solvent, and a MOF was manufactured according to the batch method by: by placing these materials in a thin-film spin-type high-speed mixer (from PRIMIX Corporation); recovering the mixture from the mixer; and introducing the mixture into the mixer again. The obtained MOF was washed with methanol and dried in a vacuum dryer at 50°C for a given amount of time. The obtained MOF was analyzed by X-ray diffraction (XRD), and it was determined to be $Cu_3(BTC)_2 \cdot H_2O$. The XRD measurement conditions are as shown below.

X-Ray Diffraction Measurement Conditions:

- Device used: MiniFlex from Rigaku Corporation.
- Tube: CuK$\alpha$ radiation.
- Measurement diffraction angle: $2\theta = 2°$ to $80°$.
- Measurement step size: 0.02°.

The median diameter $D_{50}$ of BTC used, the total amount of copper hydroxide and BTC (powder body amount), and the amount of methanol used were as shown in Table 1. Further, the circumferential speed of the mixer, the operation method, and the number of times introduction was repeated were also as shown in Table 1. Furthermore, the volume mean particle diameter of the obtained MOF, the ratio $D_{10}/D_{90}$ of the median diameter $D_{10}$ to the median diameter $D_{90}$, the volume frequency of particles having a volume mean particle diameter of less than 2 $\mu$m, and the BET specific surface area are also shown in Table 1. The conditions for measuring the volume mean particle diameter, the ratio $D_{10}/D_{90}$, the volume frequency of particles having a volume mean particle diameter of less than 2 $\mu$m, and the BET specific surface area are described below. In the present Examples, the angle of rest and compression degree were not measured, so these items are shown as "-" in the Table.

Conditions for Measuring Volume Mean Particle Diameter:

- Device used: MT3000 from MicrotracBEL Corp.
- Pretreatment conditions: ultrasonic output: 40 W; ultrasonic time: 180 seconds; degassing treatment.
- Particle size distribution: volume basis.
- Particle refractive index: 1.81.
- Measurement solvent (refractive index): water (1.333).

Conditions for Measuring Ratio $D_{10}/D_{90}$

- Device used: MT3000 from MicrotracBEL Corp.
- Pretreatment conditions: ultrasonic output: 40 W; ultrasonic time: 180 seconds; degassing treatment.
- Particle size distribution: volume basis.
- Particle refractive index: 1.81.
- Measurement solvent (refractive index): water (1.333).

Conditions for Measuring Volume Frequency of Particles Having Volume Mean Particle Diameter of Less Than 2 $\mu$m:

- Device used: MT3000 from MicrotracBEL Corp.
- Pretreatment conditions: ultrasonic output: 40 W; ultrasonic time: 180 seconds; degassing treatment.
- Particle size distribution: volume basis.
- Particle refractive index: 1.81.
- Measurement solvent (refractive index): water (1.333).

BET Specific Surface Area:

- Device used: BELSORP-mini from MicrotracBEL Corp.
- Pretreatment conditions: temperature: 150°C; retention time: 3 hours; vacuum deaeration treatment.
- Adsorbate: nitrogen.
- Adsorption temperature: 77.35 K.
- Analysis condition: BET method (type I; ISO 9277).

Examples 3 to 7:

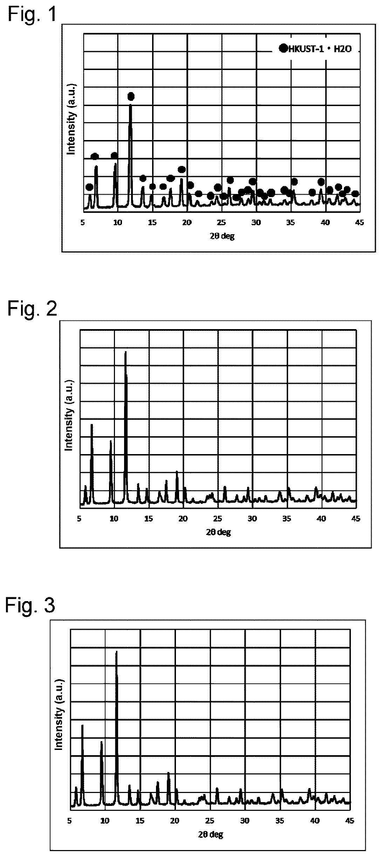

A MOF was manufactured in the same manner as in Example 1 except that copper hydroxide ($Cu(OH)_2$) was used as a metal ion donor, trimesic acid (BTC) was used as a multi dentate ligand, methanol was used as a solvent, and the circulating method (continuous method) was conducted by placing these materials in a thin-film spin-type high-speed mixer (from PRIMIX Corporation), and continuously re-introducing the recovered mixture to the upper part of the mixer, and then the obtained MOF was washed and dried. The obtained MOF was analyzed by XRD (see Fig. 1), and it was determined to be $Cu_3(BTC)_2 \cdot H_2O$. Fig. 1 shows the positions of peaks used for determining $Cu_3(BTC)_2 \cdot H_2O$. The median diameter $D_{50}$ of BTC used, the total amount of copper hydroxide and BTC, and the amount of methanol used were as shown in Table 1. Further, the circumferential speed of the mixer, the operation method, and the number of times introduction was repeated were also as shown in Table 1. Furthermore, the volume mean particle diameter of the obtained MOF, the ratio $D_{10}/D_{90}$ of the median diameter $D_{10}$ to the median diameter $D_{90}$, the volume frequency of particles having a volume mean particle diameter of less than 2 $\mu$m, the angle of rest, the compression degree, and the BET specific surface area are also shown in Table 1. The "-" in the Table shows items that were not measured. The methods for measuring the angle of rest and compression degree are described below.

Method for Measuring Angle of Rest:

Measurement was conducted according to the measurement method as described above.

Method for Measuring Compression Degree:

Measurement was conducted according to the measurement method as described above.

Comparative Examples 1 and 2:

A MOF was manufactured in the same manner as in Example 1 except that copper hydroxide ($Cu(OH)_2$) was used as a metal ion donor, trimesic acid (BTC) was used as a multidentate ligand, methanol was used as a solvent, and these materials were placed in a thin-film spin-type high-speed mixer (from PRIMIX Corporation) once, and then the obtained MOF was washed and dried.

The obtained MOF was analyzed by XRD (see Fig. 2), and it was determined to be $Cu_3(BTC)_2 \cdot H_2O$. The peaks used for determination were the same as the peaks shown in Fig. 1. The median diameter $D_{50}$ of BTC used, the total amount of copper hydroxide and BTC, and the amount of methanol used were as shown in Table 1. Further, the circumferential speed of the mixer, the operation method, and the number of times introduction was repeated were also as shown in Table 1. Furthermore, the volume mean particle diameter of the obtained MOF, the ratio $D_{10}/D_{90}$ of the median diameter $D_{10}$ to the median diameter $D_{90}$, the volume frequency of particles having a volume mean particle diameter of less than 2 $\mu$m, the angle of rest, the compression degree, and the BET specific surface area are also shown in Table 1.

Examples 8 to 11:

A MOF was manufactured in the same manner as in Example 1 except that the median diameter $D_{50}$ of trimesic acid (BTC) used as a multidentate ligand was as shown in Table 2, and the circulating method (continuous method) was conducted by placing BTC, together with copper oxide ($Cu(OH)_2$) and methanol, in a thin-film spin-type high-speed mixer (from PRIMIX Corporation) and continuously re-introducing the recovered mixture to the upper part of the mixer.

The obtained MOF was analyzed by XRD (see Fig. 3), and it was determined to be $Cu_3(BTC)_2 \cdot H_2O$. The peaks used for determination were the same as the peaks shown in Fig. 1. The median diameter $D_{50}$ of BTC used, the total amount of copper hydroxide and BTC, and the amount of methanol used were as shown in Table 2. Further, the circumferential speed of the mixer and the number of times introduction was repeated were also as shown in Table 2. Furthermore, the volume mean particle diameter of the obtained MOF, the ratio $D_{10}/D_{90}$, the volume frequency of particles having a volume mean particle diameter of less than 2 $\mu$m, the angle of rest, the compression degree, and the BET specific surface area are also shown in Table 2.

[Table 1]

| | Manufacturing conditions | | | | | | | | | | Quality | | | | | | |
| | Raw materials | | | | | | | Synthesis | | | MOF | | | | | | |
| | Metal type | Metal donor $D_{50}$ (μm) | Ligand | Ligand $D_{50}$ (μm) | Solvent type | Solvent amount (g) | Powder body amount (g) | Operation method | Circumferential speed (m/s) | Number of times of introduction | Volume mean particle diameter (μm) | $D_{10}/-D_{90}$ | Volume frequency (%) | Angle of rest (degrees) | Compression degree (%) | BET ($m^2$/g) | XRD |
|---|---|---|---|---|---|---|---|---|---|---|---|---|---|---|---|---|---|
| Example 1 | $Cu(OH)_2$ | 15.5 | BTC | 111.8 | Methanol | 36000 | 6000 | Batch | 30 | 2 | 3.2 | 0.42 | 11.2 | · | · | 1841 | $Cu_3(BTC·H_2O)$ |
| Example 2 | $Cu(OH)_2$ | 15.5 | BTC | 111.8 | Methanol | 36000 | 6000 | Batch | 30 | 3 | 2.9 | 0.42 | 18.2 | · | · | 1782 | $Cu_3(BTC)_2$ $H_2O$ |
| Example 3 | $Cu(OH)_2$ | 15.5 | BTC | 111.8 | Methanol | 36000 | 6000 | Continuous | 30 | 3 | 3.5 | 0.43 | 5.8 | · | · | 1851 | $Cu_3(BTC·H_2O)$ |
| Example 4 | $Cu(OH)_2$ | 15.5 | BTC | 70.8 | Methanol | 36000 | 6000 | Continuous | 50 | 3 | 3.0 | 0.37 | 14.5 | 44 | 44 | 1844 | $Cu_3(BTC.H_2O)$ |
| Example 5 | $Cu(OH)_2$ | 15.5 | BTC | 70.8 | Methanol | 36000 | 15000 | Continuous | 30 | 4 | 4.9 | 0.40 | 1.2 | - | - | - | $Cu_3(BTC·H_2O)$ |
| Example 6 | $Cu(OH)_2$ | 15.5 | BTC | 70.8 | Methanol | 36000 | 15000 | Continuous | 30 | 5 | 4.9 | 0.40 | 1.3 | - | - | - | $Cu_3(BTC·H_2O)$ |
| Example 7 | $Cu(OH)_2$ | 15.5 | BTC | 70.8 | Methanol | 36000 | 15000 | Continuous | 30 | 6 | 5.1 | 0.37 | 1.5 | - | - | 1770 | $Cu_3(BTC·H_2O)$ |
| Comparative Example 1 | $Cu(OH)_2$ | 15.5 | BTC | 70.8 | Methanol | 36000 | 15000 | Continuous | 30 | 1 | 157.0 | 0.08 | 0.0 | - | - | 89 | $Cu(OH)_2$+ $Cu_3$ $(BTC·H_2O)$ |
| Comparative Example 2 | $Cu(OH)_2$ | 15.5 | BTC | 70.8 | Methanol | 200 | 100 | Batch | 30 | 1 | 3.0 | 0.30 | 24.1 | 42 | 45 | 1604 | $Cu_3$ $(BTC)_2·H_2O$ |

[Table 2]

| | Manufacturing conditions | | | | | | | | | Quality | | | | | | |
| | Raw materials | | | | | | | Synthesis | | MOF | | | | | | |
| | Metal type | Metal donor $D_{50}$ ($\mu$m) | Ligand | Ligand $D_{50}$ ($\mu$m) | Solvent type | Solvent amount (g) | Powder body amount (g) | Number of times of introduction | Circumferential speed m/s | Volume mean particle diameter ($\mu$m) | DD109 | Volume frequency (%) | Angle of rest (degrees) | Compression degree (%) | BET ($m^2$/g) | XRD |
| Example 8 | $Cu(OH)_2$ | 15.0 | BTC | 26.0 | Methanol | 36000 | 15000 | 1 | 30 | 3.1 | 0.43 | 11.7 | · | · | 1679 | $Cu_3(BTC)_2 \cdot H_2O$ |
| Example 9 | $Cu(OH)_2$ | 15.0 | BTC | 26.0 | Methanol | 36000 | 15000 | 2 | 30 | 3.6 | 0.45 | 3.8 | · | · | 1761 | $Cu_3(BTC) \cdot H_2O$ |
| Example 10 | $Cu(OH)_2$ | 15.0 | BTC | 26.0 | Methanol | 36000 | 15000 | 3 | 30 | 3.5 | 0.45 | 4.7 | · | · | 1788 | $Cu_3(BTC)_2 \cdot H_2O$ |
| Example 11 | $Cu(OH)_2$ | 15.0 | BTC | 26.0 | Methanol | 36000 | 15000 | 4 | 30 | 3.5 | 0.45 | 4.5 | 36 | 29 | 1724 | $Cu_3(BTC) \cdot H_2O$ |

Tables 1 and 2 clearly show that the MOFs of the Examples all have a volume mean particle diameter of from 1.0 to 30.0 $\mu$m, and the ratio $D_{10}/D_{90}$ of the median diameter $D_{10}$ to the median diameter $D_{90}$ is from 0.35 to 1.00.

Industrial Applicability

The present invention can provide a MOF constituted by fine particles and having a sharp particle size distribution.

**Claims**

1. A metal organic framework comprising

   a volume mean particle diameter of from 1.0 to 30.0 $\mu$m, and
   a ratio $D_{10}/D_{90}$, which is a ratio of a median diameter $D_{10}$ to a median diameter $D_{90}$, of from 0.35 to 1.00.

2. The metal organic framework according to claim 1, wherein a volume frequency of particles having a particle diameter of less than 2 $\mu$m is 20% or less.

3. The metal organic framework according to claim 1 or 2, comprising an angle of rest of 50° or less.

4. The metal organic framework according to claim 1 or 2, comprising a compression degree of 50% or less.

5. The metal organic framework according to claim 1 or 2, comprising at least one type of metal ion selected from alkali metal ions, alkaline-earth metal ions, transition metal ions, Group 13 element ions, and rare-earth element ions.

6. The metal organic framework according to claim 5, wherein the metal ion includes at least one type of metal ion selected from a copper ion, an iron ion, a zinc ion, a zirconium ion, an aluminum ion, a cobalt ion, a magnesium ion, a calcium ion, a nickel ion, a chromium ion, a hafnium ion, and a lanthanum ion.

7. The metal organic framework according to claim 1 or 2, comprising at least one of organic compound selected from trimesic acid (BTC), terephthalic acid (BDC), isophthalic acid, 2-methylimidazole, 2-5-dihydroxyterephthalic acid (dobdc or DHTA), 4,4'-dioxido-3,3'-biphenyldicarboxylate (dobpdc), fumaric acid, formic acid, 1,3,5-tris(4-carboxyphenyl)benzene (BTB), 4,4'-biphenyldicarboxylic acid (BPDC), oxalic acid, 1,2,4-triazole, and 3,5-pyrazole dicarboxylic acid (H3PDC).

8. The metal organic framework according to claim 1 or 2, comprising a BET specific surface area of from 100 to 10000 $m^2$/g.

9. A method for manufacturing a metal organic framework, comprising

   a centrifuging-shearing step of simultaneously applying centrifugal force and shearing force to a blend comprising a metal ion donor, a multidentate ligand, and a solvent, and
   a step of recovering the blend subjected to the centrifuging-shearing step and subjecting the recovered blend to the centrifuging-shearing step again.

10. The method for manufacturing a metal organic framework according to claim 9, wherein a number of times the centrifuging-shearing step is conducted is from 3 to 10 times.

11. A method for manufacturing a metal organic framework, comprising a centrifuging-shearing step of simultaneously applying centrifugal force and shearing force to a blend comprising a metal ion donor, a multidentate ligand, and a solvent, wherein a median diameter $D_{50}$ of the multidentate ligand is from 0.1 to 50.0 $\mu$m.

12. The method for manufacturing a metal organic framework according to claim 11, wherein a number of times the centrifuging-shearing step is conducted is from 1 to 10 times.

13. The method for manufacturing a metal organic framework according to claim 9 or 11, wherein

the centrifuging-shearing step uses a reaction vessel including a rotary blade, and
a circumferential speed of the rotary blade is from 10 to 100 m/s.

**Amended claims under Art. 19.1 PCT**

1.  (Amended) A metal organic framework comprising

    a volume mean particle diameter of from 1.0 to 30.0 $\mu$m, and a ratio $D_{10}/D_{90}$, which is a ratio of a median diameter $D_{10}$ to a median diameter $D_{90}$, of from 0.35 to 1.00, wherein
    a volume frequency of particles having a particle diameter of less than 2 $\mu$m is 20% or less.

2.  (Cancelled)

3.  (Amended) The metal organic framework according to claim 1, comprising
    an angle of rest of 50° or less.

4.  (Amended) The metal organic framework according to claim 1, comprising
    a compression degree of 50% or less.

5.  (Amended) The metal organic framework according to claim 1, comprising
    at least one type of metal ion selected from alkali metal ions, alkaline-earth metal ions, transition metal ions, Group 13 element ions, and rare-earth element ions.

6.  The metal organic framework according to claim 5, wherein
    the metal ion includes at least one type of metal ion selected from a copper ion, an iron ion, a zinc ion, a zirconium ion, an aluminum ion, a cobalt ion, a magnesium ion, a calcium ion, a nickel ion, a chromium ion, a hafnium ion, and a lanthanum ion.

7.  (Amended) The metal organic framework according to claim 1, comprising
    at least one type of organic compound selected from trimesic acid (BTC), terephthalic acid (BDC), isophthalic acid, 2-methylimidazole, 2-5-dihydroxyterephthalic acid (dobdc or DHTA), 4,4'-dioxido-3,3'-biphenyldicarboxylate (dobpdc), fumaric acid, formic acid, 1,3,5-tris(4-carboxyphenyl)benzene (BTB), 4,4'-biphenyldicarboxylic acid (BPDC), oxalic acid, 1,2,4-triazole, and 3,5-pyrazole dicarboxylic acid (H3PDC).

8.  (Amended) The metal organic framework according to claim 1, comprising
    a BET specific surface area of from 100 to 10000 m$^2$/g.

9.  A method for manufacturing a metal organic framework, comprising

    a centrifuging-shearing step of simultaneously applying centrifugal force and shearing force to a blend comprising a metal ion donor, a multidentate ligand, and a solvent, and
    a step of recovering the blend subjected to the centrifuging-shearing step and subjecting the recovered blend to the centrifuging-shearing step again.

10. The method for manufacturing a metal organic framework according to claim 9, wherein
    a number of times the centrifuging-shearing step is conducted is from 3 to 10 times.

11. A method for manufacturing a metal organic framework, comprising

    a centrifuging-shearing step of simultaneously applying centrifugal force and shearing force to a blend comprising a metal ion donor, a multidentate ligand, and a solvent,
    wherein a median diameter $D_{50}$ of the multidentate ligand is from 0.1 to 50.0 $\mu$m.

12. The method for manufacturing a metal organic framework according to claim 11, wherein
    a number of times the centrifuging-shearing step is conducted is from 1 to 10 times.

13. The method for manufacturing a metal organic framework according to claim 9 or 11, wherein

the centrifuging-shearing step uses a reaction vessel including a rotary blade, and a circumferential speed of the rotary blade is from 10 to 100 m/s.

Fig. 1

Fig. 2

Fig. 3

TRANSLATION

**INTERNATIONAL SEARCH REPORT**

| International application No. |
|---|
| **PCT/JP2023/024708** |

| A. | CLASSIFICATION OF SUBJECT MATTER |
|---|---|

*C07C 63/307*(2006.01)i; *C07F 1/08*(2006.01)i
FI:  C07C63/307; C07F1/08 Z CSP

According to International Patent Classification (IPC) or to both national classification and IPC

| B. | FIELDS SEARCHED |
|---|---|

Minimum documentation searched (classification system followed by classification symbols)

C07C63/307; C07F1/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2018-118929 A (SUMITOMO BAKELITE CO., LTD.) 02 August 2018 (2018-08-02) claims, examples | 1, 3-8 |
| Y | claims, examples | 11-13 |
| X | WO 2020/230820 A1 (ATOMIS INC.) 19 November 2020 (2020-11-19) claims, examples, figures | 9-10 |
| Y | claims, examples, figures | 11-13 |
| A | JP 2017-144397 A (SEKISUI CHEMICAL CO., LTD.) 24 August 2017 (2017-08-24) claims, examples | 1-13 |
| A | CN 109718727 A (NORTHWESTERN UNIVERSITY) 07 May 2019 (2019-05-07) claims, examples | 1-13 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **04 September 2023** | **19 September 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
**Information on patent family members**

International application No.

**PCT/JP2023/024708**

| Patent document cited in search report | | | Publication date (day/month/year) | Patent family member(s) | | | Publication date (day/month/year) |
|---|---|---|---|---|---|---|---|
| JP | 2018-118929 | A | 02 August 2018 | (Family: none) | | | |
| WO | 2020/230820 | A1 | 19 November 2020 | US | 2022/0220129 | A1 | |
| | | | | claims, examples | | | |
| | | | | EP | 3971160 | A1 | |
| | | | | CN | 113825738 | A | |
| JP | 2017-144397 | A | 24 August 2017 | (Family: none) | | | |
| CN | 109718727 | A | 07 May 2019 | (Family: none) | | | |

Form PCT/ISA/210 (patent family annex) (January 2015)

**INTERNATIONAL SEARCH REPORT**

International application No.

**PCT/JP2023/024708**

| Box No. III | Observations where unity of invention is lacking (Continuation of item 3 of first sheet) |
|---|---|

This International Searching Authority found multiple inventions in this international application, as follows:

The matter common to the inventions in claims 1-13 can be considered to be an organic metal structure.

However, JP 2018-118989A (SUMITOMO BAKELITE CO., LTD.) sets forth an organic metal structure, and an organic metal structure therefore cannot be considered a special technical feature as set forth in PCT Rule 13.2.

In consideration of the foregoing, the present international application encompasses the following three inventions.
Invention 1: Claims 1-8 (metal organic structure having a specific parameter)
Invention 2: Claims 9-10 (method of manufacturing the organic metal structure)
Invention 3: Claims 11-13 (method of manufacturing the organic metal structure having the specific parameter)

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**  ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3971160 A1
- JP 2007125454 A